(19) Europäisches Patentamt
European Patent Office
Office européen des brevets

(11) Numéro de publication: **0 314 582 B1**

(12) **FASCICULE DE BREVET EUROPEEN**

(45) Date de publication de fascicule du brevet: **10.02.93**

(51) Int. Cl.[5]: **B01J 29/04**, C07C 37/60, C07C 39/08, C07C 43/23, C07C 43/295

(21) Numéro de dépôt: **88420350.6**

(22) Date de dépôt: **14.10.88**

Le dossier contient des informations techniques présentées postérieurement au dépôt de la demande et ne figurant pas dans le présent fascicule.

# (54) Procédé d'hydroxylation de phénols et d'éthers de phénols.

(30) Priorité: **29.10.87 FR 8715247**

(43) Date de publication de la demande: **03.05.89 Bulletin 89/18**

(45) Mention de la délivrance du brevet: **10.02.93 Bulletin 93/06**

(84) Etats contractants désignés: **AT BE CH DE ES FR GB GR IT LI LU NL SE**

(56) Documents cités:
**EP-A- 0 200 260**
**EP-A- 0 292 363**
**WO-A-85/04853**
**FR-A- 2 429 182**
**FR-A- 2 471 950**
**GB-A- 2 116 974**
**US-A- 3 481 989**

**CHEMICAL ABSTRACTS, tome 105, n. 10, 08.09.86, page 456. Columbus, Ohio, US; F. Gatti "Effect of aluminium content on the ZSM-5 zeolite crystallization in the presence of alkanolamine"**

(73) Titulaire: **RHONE-POULENC CHIMIE**
**25, quai Paul Doumer**
**F-92408 Courbevoie Cédex(FR)**

(72) Inventeur: **Costantini, Michel**
**10, rue du Docteur Bonhomme**
**F-69003 Lyon(FR)**
Inventeur: **Popa, Jean-Michel**
**2, rue Roger Breton**
**F-93700 Drancy(FR)**
Inventeur: **Gubelmann, Michel**
**39, Boulevard des Belges**
**F-69006 Lyon(FR)**

(74) Mandataire: **Dutruc-Rosset, Marie-Claude et al**
**RHONE-POULENC CHIMIE Direction des Brevets 25, Ouai Paul Doumer**
**F-92408 Courbevoie Cédex(FR)**

EP 0 314 582 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un procédé d'hydroxylation de phénols ou d'éthers de phénols par le peroxyde d'hydrogène.

L'hydroxylation du phénol ou de phénols substitués, par le peroxyde d'hydrogène, pour préparer des diphénols est une réaction connue.

Le brevet français n° 69-45467 publié sous le n° 2 071 464 a décrit un procédé dans lequel la réaction est catalysée par un acide fort, tel que par exemple l'acide perchlorique ou l'acide sulfurique.

Le brevet allemand n° 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, qui se forment le plus souvent lors de l'élimination des catalyseurs acides dissous.

Ainsi, la demande de brevet français n° 81-17023 (publiée sous le n° 2 489 816) préconise l'emploi de silicalite de titane comme catalyseur hétérogène de l'hydroxylation de composés aromatiques par le peroxyde d'hydrogène : ledit procédé pouvant être également conduit en présence d'acétone (GB-A 2 116 974).

En fait, cette catalyse présente de grandes difficultés de reproductibilité. En outre la fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n° 200 260, d'utiliser des agglomérats de ces fines particules de silicalite de titane.

Il s'avère cependant que l'on recherche encore une catalyse hétérogène de la réaction d'hydroxylation des phénols ou éthers de phénols par le peroxyde d'hydrogène, qui puisse être utilisée de manière industrielle dans des conditions économiquement acceptables. C'est précisément l'objet de la présente invention.

L'invention consiste donc en un procédé d'hydroxylation de phénols ou éthers de phénols de formule générale (I) :

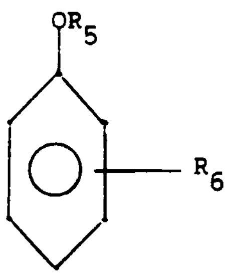

(I)

dans laquelle :

- $R_5$ représente un atome d'hydrogène, un groupement méthyle, un groupement éthyle ou un groupement phényle,
- $R_6$ représente un atome d'hydrogène, un radical alkyle ayant 1 à 4 atomes de carbone, un radical alkoxy ayant 1 à 4 atomes de carbone, un radical phényle ou cyclohexyle ; par réaction avec le peroxyde d'hydrogène, caractérisé en ce que la réaction est conduite en présence d'une quantité efficace de zéolite de structure MFI à base d'oxyde de silicium et d'oxyde et titane et ayant après calcination la formule suivante (II) :

$Si_{(96-x)}, Ti_x O_{192}$ (II)

dans laquelle :

x est compris entre 0,1 et 6 environ.

Ces zéolites seront dénommées dans le présent texte titanozéosilites.

La titanozéosilite utilisée dans le procédé de l'invention a un système cristallin monoclinique et un diagramme de diffraction des rayons x défini dans le tableau I.

Dans ce tableau, les valeurs extrêmes des différentes équi-distances réticulaires $d_{hkl}$ sont données et correspondent aux concentrations limites de titane incorporé dans la charpente de la zéolite, ou plus précisement au rapport Ti/Si.

En effet, l'identification des titanozéosilites peut être notamment et avantageusement réalisée par l'établissement de leur diagramme de diffraction des rayons X.

Ce diagramme de diffraction peut être obtenu à l'aide d'un diffractomètre en utilisant la méthode classique des poudres avec le rayonnement $K\alpha$ du cuivre. A partir de la position des pics de diffraction représentée par l'angle $2\theta$ on calcule, par la relation de Bragg, les équi-distances réticulaires $d_{hkl}$ caractéristiques de l'échantillon. L'estimation de l'erreur de mesure $\Delta(d_{hkl})$ sur $d_{hkl}$ se calcule, en fonction de l'erreur absolue $\Delta(2\theta)$ affectée à la mesure de $2\theta$ , par la relation de Bragg. Une erreur absolue $\Delta(2\theta)$ égale à ± 0,2° est couramment admise. L'intensité relative $I/I_0$ affectée à chaque valeur de $d_{hkl}$ est estimée à partir de la hauteur du pic de diffraction correspondant. On utilise souvent une échelle de symboles pour caractériser cette intensité : FF = très fort, F = fort, mF = moyen à fort, m = moyen, mf = moyen à faible, f = faible, ff = très faible.

**TABLEAU I**

**diagramme de diffraction des rayons X**

| Valeurs extrêmes des $d_{hkl}$ (nm) | $I/I_o$ | Valeurs extrêmes des $d_{hkl}$ (nm) | $I/I_o$ |
|---|---|---|---|
| 1,110-1,128 | F-FF | 0,3785-0,3845 | mF |
| 0,991-1,012 | F-FF | 0,3735-0,3795 | m |
| 0,972-0,986 | f | 0,3715-0,3775 | m |
| 0,895-0,906 | ff | 0,3705-0,3765 | m |
| 0,803-0,813 | ff | 0,3645-0,3700 | f |
| 0,741-0,753 | ff (large) | 0,3610-0,3670 | f |
| 0,704-0,715 | ff (large) | 0,3470-0,3525 | ff |
| 0,666-0,678 | f | 0,3430-0,3485 | f(f) |
| 0,632-0,643 | f | 0,3415-0,3470 | f(f) |
| 0,595-0,605 | mf | 0,3385-0,3439 | ff |
| 0,589-0,598 | f | 0,3341-0,3394 | f(f) |
| 0,568-0,577 | mf | 0,3290-0,3345 | f (large) |
| 0,565-0,574 | f (épaulement) | 0,3240-0,3292 | f |
| 0,555-0,564 | f | 0,3045-0,3099 | f(f) |
| 0,534-0,543 | f(f) | 0,3020-0,3068 | f |
| 0,531-0,539 | f(f) | 0,2978-0,3026 | f |
| 0,510-0,518 | ff | 0,2952-0,2999 | ff (épaulement) |
| 0,502-0,508 | ff | 0,2944-0,2991 | f |
| 0,496-0,504 | mf | 0,2914-0,2961 | ff |
| 0,485-0,493 | ff | 0,2852-0,2898 | ff (large) |
| 0,468-0,476 | ff | 0,2774-0,2818 | ff |
| 0,459-0,466 | f | 0,2722-0,2766 | ff (large) |
| 0,444-0,451 | f | 0,2676-0,2720 | ff |
| 0,433-0,441 | f | 0,2606-0,2648 | ff |
| 0,423-0,431 | f | 0,2586-0,2627 | ff |
| 0,4055-0,4125 | ff | 0,2544-0,2585 | ff (large) |
| 0,3985-0,4045 | f | 0,2508-0,2548 | ff |
| 0,3835-0,3905 | F | 0,2478-0,2518 | f |
| 0,3805-0,3865 | mF | | |

Généralement la titanozéosilite contient du fluor, la concentration en fluor est comprise entre 0,01 et 0,8 % en poids après calcination.

Toutefois, le fluor peut être éliminé sans pour cela modifier la structure de la titanozéosilite utilisée dans l' invention.

La titanozéosilite servant de catalyseur dans le présent procédé fait l'objet d'une demande parallèle, EP-A-0 292 363. Elle peut être synthétisée de la manière suivante :

(i) préparation d'un mélange réactionnel en milieu aqueux contenant au moins une source d'oxyde de silicium, une source d'oxyde de titane, des ions fluorures, et un agent structurant, le pH de ce mélange réactionnel étant compris entre 1,5 environ et 10,5 environ,

(ii) cristallisation de ce mélange réactionnel et la récupération du précipité cristallin, et

(iii) calcination de celui-ci à une température supérieure à 450 °C.

Le diagramme de diffraction des rayons X donné dans le tableau I est celui d'une titanozéosilite soumise à une calcination comme indiqué ci-dessus.

L'emploi d'ions fluorures dans le milieu réactionnel, qui jouent le rôle d'agent mobilisateur, permet d'obtenir une solubilisation des espèces T (Si et Ti) dans un milieu ayant un pH inférieur à 10. Ainsi, il est possible d'utiliser comme cations de compensation des ions $NH_4^+$, qui pourront être éliminés totalement, si on le désire, lors de la calcination.

De plus, la cristallisation s'effectuant dans un milieu à pH inférieur à 10, la vitesse de nucléation est plus lente. Ainsi, il est possible d'obtenir des cristaux de titanozéosilites contrôlés par pilotage de la vitesse de nucléation.

Les rapports molaires entre les différentes espèces dans le milieu réactionnel sont compris pour Ti/Si entre 1,5 environ et 0,002 environ, F/Si entre 10 et 0,04 environ, $H_2O$/Si entre 400 environ et 4 environ et pour l'agent structurant par rapport à l'espèce silicium entre 2 environ et 0,02 environ.

Avantageusement, le rapport molaire Ti/Si est compris entre 1 et 0,01, F/Si entre 6 et 0,06, $H_2O$/Si entre 100 et 6 et entre l'agent structurant et l'espèce silicium entre 1 et 0,04.

De nombreuses sources de silice peuvent être utilisées. On peut citer, à titre d'exemple, les silices sous formes d'hydrogels, d'aérogels, de suspensions colloïdales, les silices résultant de la précipitation à partir de solutions de silicates solubles ou de l'hydrolyse d'esters siliciques comme $Si(OC_2H_5)_4$ ou de complexes comme $Na_2SiF_6$, les silices préparées par des traitements d'extraction et d'activation de composés cristallisés naturels ou synthétiques comme les silicates d'aluminium, les aluminosilicates, les argiles. On peut également utiliser des composés du silicium tétravalent hydrolysables tels que les halogénures de silicium.

Parmi les sources d'oxyde de titane, on peut citer, à titre d'exemple, les oxydes et les hydroxydes de titane, cristallisés ou amorphes, les composés du titane tétravalent pouvant être hydrolysés tels les halogénures ($TiCl_4$), les alcoolates, les sels solubles de titane tels $TiOSO_4$, $(NH_4)_2$ $TiO(C_2O_4)_2$.

Il est également possible d'utiliser comme sources de silice ou d'oxyde de titane, des composés comprenant les éléments Si et Ti tels que, par exemple, des verres ou gels à base des oxydes de ces deux éléments.

Les sources de silice et d'oxyde de titane peuvent être engagées sous forme soluble ou de solides pulvérulents mais également sous forme d'agglomérats tels que, par exemple, pastilles, extrudés pouvant être transformés en titanozéosilite de structure désirée sans modification de forme.

Les anions fluorures peuvent être introduits sous forme d'acide fluorhydrique, de sels tels que, par exemple, $NH_4F$, $NH_4HF_2$, $NH(C_3H_7)_3F$, $N(C_3H_7)_4F$, de composés hydrolysables libérant les anions fluorures dans le milieu réactionnel, tels que, par exemple $SiF_4$, $(NH_4)_2SiF_6$, $(NH_4)_2TiF_6$ ou analogues.

Le fluorure d'ammonium ou le fluorure d'ammonium acide sont des sels préférés. En effet ces sels sont très solubles et n'apportent aucun élément indésirable et, de plus, ils sont facilement éliminables en fin de cristallisation.

Les agents structurants convenables pour la préparation des titanozéosiliteS sont :

- les amines de formule (III) :

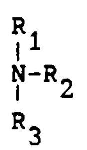

(III)

dans laquelle :

$R_1$, $R_2$, $R_3$ identiques ou différents représentent un groupe alkyle, de préférence un groupe propyle ou butyle.

- les ammonium quaternaires de formule (IV) :

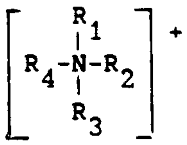

(IV)

dans laquelle :

$R_1$, $R_2$, $R_3$, $R_4$ identiques ou différents représentent des groups alkyles, de préférence des groupes propyles ou butyles.

- les composés de formules III et IV dans lesquelles l'azote a été remplacé par un atome de phosphore.

Les agents structurants sont de préférence la tripropylamine ou les composés qui peuvent fournir des cations tétrapropylammonium.

Avantageusement, l'agent structurant est ajouté au mélange réactionnel sous forme d'un sel d'une amine ou d'un ammonium quaternaire fournissant les cations ci-dessus mentionnés.

Le mélange réactionnel peut comprendre un agent co-mobilisateur du titane tétravalent dans un rapport molaire par rapport au silicium compris entre 3 et 0,01 et avantageusement entre 2 et 0,04.

Les agents co-mobilisateurs convenables pour la préparation des titanozéosilites utilisées dans le présent procédé sont, par exemple, l'acide oxalique et ses sels, l'acide tartrique et ses sels.

La cristallisation de la titanozéosilite peut être obtenue par chauffage du mélange réactionnel à une température comprise entre 50 ° C environ et 240 ° C environ, de préférence entre 75 ° C et 225 ° C pendant le temps nécessaire à la cristallisation, selon un mode opératoire classique de synthèse des zéolites et connu de l'homme du métier. A titre indicatif, la durée de chauffage peut être comprise entre 6 heures et 500 heures environ.

Ce chauffage et cette cristallisation sont réalisés de préférence dans un récipient ou autoclave revêtu d'une couche telle que, par exemple, le polytétrafluoroéthane.

Le mélange réactionnel peut être agité ou non.

Après cristallisation, le précipité obtenu est recueilli, par exemple, par filtration.

Ce précipité est ensuite chauffé après un séchage éventuel, à une température supérieure à 450 ° C, de préférence supérieure à 500 ° C, afin de décomposer par calcination ou décomposition thermique les espèces organiques contenues dans le précipité, telles que, par exemple, l'agent structurant, les cations de compensation ($NH_4^+$).

Les phenols et éthers de phénols qui sont utilisés de préférence dans le procédé de l'invention sont les composés de formule (I) dans laquelle $R_5$ représente un atome d'hydrogène, un groupement méthyle ou un groupement éthyle, et $R_6$ représente un atome d'hydrogène, un groupement méthyle, éthyle ou tertiobutyle, un groupement méthoxy ou éthoxy.

A titre d'exemple non limitatifs on peut citer le phénol, l'anisole, l'orthocrésol, le métacrésol, le paracrésol, le tertiobutyl-4 phénol, le méthoxy-2 phénol, le méthoxy-4 phénol.

Le procédé selon l'invention s'applique particulièrement bien au phénol pour la préparation d'hydroquinone et de pyrocatéchine.

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvant organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl-tertiobutyléther.

Le rapport molaire composé de formule I/peroxyde d'hydrogène est généralement de 25/1 à 3/1 et préférentiellement de 20/1 à 4/1.

La quantité de titanozéosilite définie précédemment, que l'on peut mettre en oeuvre dans le présent procédé peut varier dans de très larges limites.

Lorsque l'on réalise le procédé en discontinu, le catalyseur peut représenter en poids par rapport au poids du composé de formule (I) engagé de 0,1 % à 20 %. De préférence, ce rapport pondéral sera compris entre 0,5 % et 10 %. Cependant si l'on réalise le procédé en continu, par exemple en faisant réagir un mélange de composé (I) et de solution de peroxyde d'hydrogène sur un lit fixe de catalyseur, ces rapports catalyseur/composé (I) n'ont plus de sens et, à un instant donné, on pourra avoir un excès pondéral de catalyseur par rapport au composé (I).

Il est également possible d'effectuer la réaction d'hydroxylation du composé (I) dans un solvant du

composé (I), qui soit de préférence miscible ou partiellement miscible à l'eau.

A titre d'exemples non limitatifs de tels solvants, on peut citer l'eau ; les alcools comme le méthanol, le tertiobutanol, l'isopropanol ou l'éthanol ; les cétones comme l'acétone ou la méthylisobutylcétone ; les nitriles comme l'acétonitrile ; les acides carboxyliques comme l'acide acétique ; les esters comme l'acétate de propyle ; les éthers comme le méthyl-tertiobutyléther ; des solvants aprotiques polaires comme le dioxyde de tétrahydrothiophène (sulfolane), le carbonate d'éthylène glycol, le carbonate de propylène glycol, la N-méthylpyrrolidone.

La température à laquelle est conduite la réaction est généralement comprise entre 45° et 160°C sous pression atmosphérique.

On peut également opérer à plus haute température et sous pression supérieure à la pression atmosphérique.

Les exemples qui suivent illustrent l'invention.

EXEMPLE 1 : PREPARATION DES CATALYSEURS 1a et 1b

Catalyseur 1a

On réalise un mélange réactionnel selon le mode opératoire suivant :

On hydrolyse 5,45 g de $Ti(OC_4H_9)_4$ dans 100 cm3 d'eau, en agitant ce mélange pendant 6 heures. Le précipité obtenu est filtré, puis dissout à chaud dans 107 cm3 d'eau en présence de 4,03 g d'acide oxalique ($C_2H_2O_4$, 2 $H_2O$). A cette solution d'oxalate de titane, on ajoute une solution contenant 10,65 g de bromure de tétrapropylammonium (TPA-Br), 2,96 g de fluorure d'ammonium ($NH_4F$), 0,19 g de germes de zéolite MFI et 9,6 g de silice type Aérosil 130.

On agite pendant 15 minutes environ.

La composition molaire ramenée à une mole de silice du mélange réactionnel est la suivante :

1 $SiO_2$ ; 0,1 $TiO_2$ ; 0,2 $C_2H_2O_4$ ; 0,25 TPA-Br ; 0,5 $NH_4F$ ; 37 $H_2O$.

Le mélange réactionnel est ensuite cristallisé dans un autoclave revêtu intérieurement par du PTFE par chauffage à 170°C pendant 7 jours sans agitation.

Après cristallisation, la phase solide est séparée par filtration, lavée à l'eau et séchée à 80°C. Après calcination à 550°C pendant 4 heures, la phase solide est identifiée par son spectre de diffraction des rayons X. On constate qu'elle contient plus de 90 % de titanozéosilite.

La titanozéosilite se présent sous forme de cristaux longs et fins (avec des petites billes) de 15 à 20 micromètres.

L'analyse chimique effectuée sur le produit de l'exemple 1a donne un rapport molaire Si/Ti global de 25. L'estimation du rapport molaire Si/Ti dans la charpente de la zéolite peut être faite à partir de la mesure des déplacements relatifs des pics de diffraction. On trouve alors une valeur égale à environ 60.

Le spectre de diffraction des rayons X de la titanozéosilite obtenue dans l'exemple 1a est indiqué ci-après.

| dhkl(nm) | I/Io |
|---|---|
| 0,462 | f |
| 0,446 | ff |
| 0,436 | f |
| 0,426 | f |
| 0,4084 | ff |
| 0,4008 | f |
| 0,3859 | F |
| 0,3826 | mF |
| 0,3806 | mF |
| 0,3759 | m |
| 0,3742 | m |
| 0,3718 | m |
| 0,3663 | f |
| 0,3627 | f |
| 0,3448 | f(f) |
| 0,3431 | f |
| 0,3396 | ff |
| 0,3357 | f(f) |
| 0,3317 | f |
| 0,3256 | f |

Catalyseur 1b

On réalise un mélange réactionnel selon le mode opératoire suivant :

On hydrolyse 2,72 g de $Ti(OC_4H_9)_4$ dans 50 cm3 d'eau, en agitant ce mélange pendant 6 heures. Le précipité obtenu est filtré, puis dissout à chaud dans 43,2 cm3 d'eau en présence de 2,02 g d'acide oxalique ($C_2H_2O_4$, 2 $H_2O$). A cette solution d'oxalate de titane, on ajoute une solution contenant 5,33 g de bromure de tétrapropylammonium (TPA-Br), 1,48 g de fluorure d'ammonium ($NH_4F$), 0,096 g de germes de zéolite MFI et 4,8 g de silice type Aérosil 130.

On agite pendant 15 minutes environ.

La composition molaire ramenée à une mole de silice du mélange réactionnel est la suivante :

1 $SiO_2$ ; 0,1 $TiO_2$ ; 0,2 $C_2H_2O_4$ ; 0,25 TPA-Br ; 0,5 $NH_4F$; 30 $H_2O$.

Le mélange réactionnel est ensuite cristallisé dans un autoclave revêtu intérieurement par du PTFE par chauffage à 200 ° C pendant 6 jours sans agitation.

Après cristallisation, la phase solide est séparée par filtration, lavée à l'eau et séchée à 70 ° C. Après calcination à 550 ° C pendant 4 heures, la phase solide est identifiée par son spectre de diffraction des rayons X. On constate qu'elle contient plus de 90 % de titanozéosilite.

La titanozéosilite se présente sous forme de cristaux allongés très fins d'environ 10 micromètres de longueur.

L'analyse chimique effectuée sur le produit de l'exemple 1b donne un rapport molaire Si/Ti global de 25. L'estimation du rapport molaire Si/Ti dans la charpente de la zéolite peut être faite à partir de la mesure des déplacements relatifs des pics de diffraction. On trouve alors une valeur égale à environ 60.

Le spectre de diffraction des rayons X de la titanozéosilite obtenue dans l'exemple 1b est le même que dans l'exemple 1a.

EXEMPLE 2

Dans un réacteur en verre pyrex de 100 cm3, muni d'une agitation centrale, d'un réfrigérant relié à un gazomètre, d'un système de chauffage régulé et d'un système d'injection, on charge après avoir préalablement purgé l'appareil à l'aide d'azote :

- 26,95 g de phénol
- 0,513 g de titanozéosilite préparée dans l'exemple 1a.

On chauffe à 80 °C sous agitation, puis on injecte en 1 heure une solution aqueuse de $H_2O_2$ à 40% en poids par volume. (0,063 mole de $H_2O_2$).

On laisse ensuite réagir pendant 2 heures supplémentaires.

Après filtration du catalyseur, on dose $H_2O_2$ non transformée par iodométrie et les diphénols par chromatographie liquide haute performance (CLHP).

On obtient les résultats suivants :

| | |
|---|---|
| - taux de transformation (TT)de $H_2O_2$ : | 24,1 % |
| - rendement en pyrocatéchine par rapport à $H_2O_2$ transformée (RT) : | 16,0 % |
| - rendement en hydroquinone par rapport à $H_2O_2$ transformée (RT) : | 25,0 % |
| - rendement total en diphénols : | 41,0 % |

EXEMPLE 3

Dans un réacteur en verre pyrex de 30 $cm^3$, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :

- 9,4 g de phénol (0,1 mole)
- 0,26 g de titanozéosilite préparée dans l'exemple 1a.

On chauffe à 80 °C sous agitation, puis on injecte une solution aqueuse de $H_2O_2$ à 70 % en poids par volume. (0,005 mole de $H_2O_2$).

On laisse ensuite réagir pendant 2 heures 30 minutes.

Après filtration du catalyseur, on dose $H_2O_2$ non transformée par iodométrie et les diphénols par chromatographie liquide haute performance (CLHP).

On obtient les résultats suivants :

| | |
|---|---|
| - taux de transformation (TT)de $H_2O_2$ : | 30,5 % |
| - rendement en pyrocatéchine par rapport à $H_2O_2$ transformée (RT) : | 24,0 % |
| - rendement en hydroquinone par rapport à $H_2O_2$ transformée (RT) : | 36,0 % |
| - rendement total en diphénols : | 60,0 % |

EXEMPLE 4

On répète l'exemple 3 avec les mêmes quantités de réactifs, mais l'essai est réalisé à 130 °C au lieu de 80 °C.

On obtient les résultats suivants :

| | |
|---|---|
| - taux de transformation (TT)de $H_2O_2$ : | 93,0 % |
| - rendement en pyrocatéchine par rapport à $H_2O_2$ transformée (RT) : | 35,5 % |
| - rendement en hydroquinone par rapport à $H_2O_2$ transformée (RT) : | 33,0 % |
| - rendement total en diphénols : | 68,5 % |

EXEMPLES 5 à 11

Dans un réacteur en verre pyrex de 30 cm3, muni d'une agitation magnétique, d'un réfrigérant relié à un gazomètre, d'un système de chauffage régulé et d'un système d'injection, on charge après avoir préalablement purgé l'appareil à l'aide d'azote :

- 4,7 g de phénol
- 0,25 g de titanozéosilite préparée dans l'exemple 1, (1a pour les exemples 5 à 9, 1b pour les exemples 10 à 11)
- 4,7 g d'un solvant (voir tableau ci-après).

On chauffe à 80 ° C sous agitation, puis on injecte une solution de $H_2O_2$ à 70 % en poids par volume (2,5 mmol).

On laisse ensuite réagir pendant 2 h 30 minutes.

Après filtration du catalyseur, on dose $H_2O_2$ non transformée par iodométrie et les diphénols par chromatographie liquide haute performance (CLHP).

Le tableau ci-après rassemble les résultats obtenus (pyrocatéchine = PC ; hydroquinone = HQ).

| EXEMPLES | SOLVANTS | TT % H2O2 | RT % HQ | RT % PC | Somme RT % |
|---|---|---|---|---|---|
| Ex. 5 | acide acétique | 70,0 | 11,0 | 8,5 | 19,5 |
| Ex. 6 | acétonitrile | 7,5 | 30,0 | 60,0 | 90,0 |
| Ex. 7 | méthyl-isobutyl-cétone | 14,0 | 13,0 | 17,5 | 30,5 |
| Ex. 8 | tertiobutanol | 6,0 | 42,0 | 52,5 | 94,5 |
| Ex. 9 | sulfolane | 28,0 | 16,0 | 20,0 | 36,0 |
| Ex. 10 | eau | 73,0 | 26,0 | 51,0 | 77,0 |
| Ex. 11 | méthyl-tertio-butyléther | 2,5 | 48,0 | 48,0 | 96,0 |

EXEMPLE 12

On répète l'exemple 10 selon le mode opératoire décrit pour les exemples 5 à 11, mais en opérant à 100 ° C (avec l'eau comme solvant).

Le traitement de l'essai et les dosages sont effectués comme dans les exemples 5 à 11.

On obtient les résultats suivants :

- taux de transformation de $H_2O_2$ : 92,5 %
- rendement en pyrocatéchine par rapport à $H_2O_2$ transformée : 39,0 %
- rendement en hydroquinone par rapport à $H_2O_2$ transformée : 39,5 %
- rendement en diphénols par rapport à $H_2O_2$ transformée : 78,5 %

**Revendications**

**1.** Procédé d'hydroxylation de phénols ou éthers de phénols de formule générale (I) :

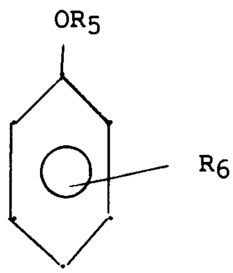

(I)

dans laquelle :

- $R_5$ représente un atome d'hydrogène, un groupement méthyle, un groupement éthyle ou un groupement phényle,
- $R_6$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace de zéolite de structure MFI à base d'oxyde de silicium et d'oxyde de titane :

- ayant après calcination la formule suivante (II) :

  $Si_{(96-x)}$, $Ti_x$ $O_{192}$ (II)

  dans laquelle :

  x est compris entre 0,1 et 6 environ
- contenant entre 0,01 et 0,8 % de fluor en poids, après calcination,
- et présentant un système cristallin monoclinique.

**2.** Procédé d'hydroxylation de phénols ou éthers de phénols de formule générale (I) :

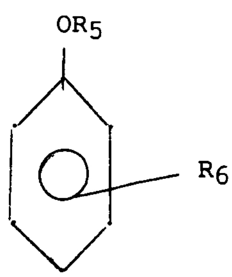

(I)

dans laquelle :

- $R_5$ représente un atome d'hydrogène, un groupement méthyle, un groupement éthyle ou un groupement phényle,
- $R_6$ représente un atome d'hydrogène, un radical alkyle ayant 1 à 4 atomes de carbone, un radical alkoxy ayant 1 à 4 atomes de carbone, un radical phényle ou cyclohexyle ;

par réaction avec le peroxyde d'hydrogène, caractérisé en ce que la réaction est conduite en présence d'une quantité efficace de zéolite de structure MFI à base d'oxyde de silicium et d'oxyde de titane ayant après calcination la formule suivante (II) :

$Si_{(96-x)}$, $Ti_x$ $O_{192}$ (II)

dans laquelle :

x est compris entre 0,1 et 6 environ obtenue par un procédé comportant les étapes suivantes :

(i) préparation d'un mélange réactionnel en milieu aqueux contenant au moins une source d'oxyde de silicium, une source d'oxyde de titane, des ions fluorures, et un agent structurant, le pH de ce mélange réactionnel étant compris entre 1,5 environ et 10,5 environ,

(ii) cristallisation de ce mélange réactionnel et la récupération du précipité cristallin, et
(iii) calcination de celui-ci à une température supérieure à 450 ° C.

**3.** Procédé selon la revendication 2 caractérisé en ce que la titanozéosilite utilisée est obtenue par un procédé comportant les étapes suivantes :
(i) préparation d'un mélange réactionnel en milieu aqueux contenant au moins une source d'oxyde de silicium, une source d'oxyde de titane, des ions fluorures, et un agent structurant, avec les rapports molaires Ti/Si compris entre 1,5 et 0,002, F/Si entre 10 et 0,04, $H_2O$/Si entre 400 et 4, agent structurant/Si entre 2 et 0,02 et un pH de mélange réactionnel étant compris entre 1,5 environ et 10,5 environ,
(ii) cristallisation de ce mélange réactionnel et la récupération du précipité cristallin, et
(iii) calcination de celui-ci à une température supérieure à 450 ° C.

**4.** Procédé selon la revendication 3, caractérisé en ce que lors de la préparation de la titanozéosilite le rapport molaire Ti/Si dans le mélange réactionnel est compris entre 1 et 0,01.

**5.** Procédé selon l'une des revendications 3 ou 4, caractérisé en ce que lors de la préparation de la titanozéosilite le rapport molaire F/Si dans le mélange réactionnel est compris entre 6 et 0,06.

**6.** Procédé selon l'une des revendications 3 à 5, caractérisé en ce que lors de la préparation de la titanozéosilite le rapport molaire $H_2O$/Si dans le mélange réactionnel est compris entre 100 et 6.

**7.** Procédé selon l'une des revendications 3 à 6, caractérisé en ce que lors de la préparation de la titanozéosilite le rapport molaire agent structurant/Si est compris entre 1 et 0,04.

**8.** Procédé selon l'une des revendications 1 à 7, caractérisé en ce que le rapport molaire phénol/peroxyde d'hydrogène est de 25/1 à 3/1 et de préférence de 20/1 à 4/1.

**9.** Procédé selon l'une des revendications 1 à 8, réalisé en discontinu, caractérisé en ce que le catalyseur représente en poids par rapport au poids du composé de formule (I) engagé de 0,1 % à 20 % et de préférence de 0,5 % à 10 %.

**10.** Procédé selon l'une des revendications 1 à 9, caractérisé en ce que le procédé est réalisé en continu sur un lit fixe de catalyseur.

**11.** Procédé selon l'une des revendications 1 à 10, caractérisé en ce que le peroxyde d'hydrogène est mis en oeuvre sous la forme d'une solution aqueuse.

**12.** Procédé selon l'une des revendications 1 à 11, caractérisé en ce que le peroxyde d'hydrogène est mis en oeuvre sous la forme d'une solution organique.

**13.** Procédé selon l'une des revendications 1 à 12, caractérisé en ce que la réaction d'hydroxylation est effectuée dans un solvant du composé de formule (I) de préférence miscible ou partiellement miscible à l'eau, tel que l'eau, un alcool, une cétone, un nitrile, un acide carboxylique, un ester, un éther ou un solvant aprotique polaire.

**14.** Procédé selon l'une des revendications 1 à 13, caractérisé en ce que la réaction d'hydroxylation est conduite à une température comprise entre 45 ° C et 160 ° C.

**15.** Procédé selon l'une des revendications 1 à 14, caractérisé en ce qu'il est appliqué aux phénols et éthers de phénol de formule (I) dans laquelle $R_5$ représente un atome d'hydrogène, un groupement méthyle ou un groupement éthyle, et $R_6$ représente un atome d'hydrogène, un groupement méthyle, éthyle ou tertiobutyle, un groupement méthoxy ou éthoxy.

**16.** Procédé selon l'une des revendications 1 à 15 caractérisé en ce qu'il est appliqué aux phénols et éthers de phénols choisi parmi le phénol, l'anisole, l'orthocrésol, le métacrésol, le paracrésol, le tertiobutyl-4 phénol, le méthoxy-2 phénol, le méthoxy-4 phénol.

## Claims

1. Process for the hydroxylation of phenols or ethers of phenols of general formula (I):

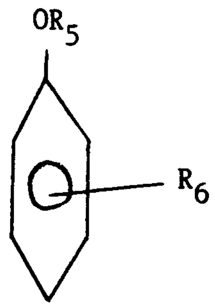

(I)

in which:
$R_5$ represents a hydrogen atom, a methyl group, an ethyl group or a phenyl group,
$R_6$ represents a hydrogen atom, an alkyl radical having 1 to 4 carbon atoms, an alkoxy radical having 1 to 4 carbon atoms, a cyclohexyl or phenyl radical,
by reaction with hydrogen peroxide, characterized in that the reaction is performed in the presence of an effective quantity of a MFI structure zeolite based on silicon dioxide and titanium dioxide,
having after calcination the following formula (II):

$S_{(96-x)}, Ti_x O_{192}$ (II)

in which:
x is between approximately 0.1 and 6,
containing between 0.01 and 0.8% by weight fluorine, after calcination,
and having a monoclinic crystalline system.

2. Process for the hydroxylation of phenols or ethers of phenols of general formula (I):

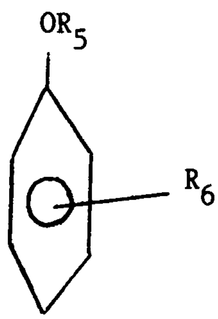

(I)

in which:
$R_5$ represents a hydrogen atom, a methyl group, an ethyl group or a phenyl group,
$R_6$ represents a hydrogen atom, an alkyl radical having 1 to 4 carbon atoms, an alkoxy radical having 1 to 4 carbon atoms, a cyclohexyl or phenyl radical,
by reaction with hydrogen peroxide, characterized in that the reaction is performed in the presence of an effective quantity of a MFI structure zeolite based on silicon dioxide and titanium dioxide,
having after calcination the following formula (II):

$S_{(96-x)}, Ti_x O_{192}$ (II)

in which
x is between approximately 0.1 and 6,
obtained by a process having the following stages:

(i) preparation of a reaction mixture in an aqueous medium containing at least one silicon dioxide source, a titanium dioxide source, fluoride ions and a structuring agent, the pH of said reaction mixture being between approximately 1.5 and approximately 10.5,
(ii) crystallization of this reaction mixture and recovery of the crystalline precipitate and
(iii) calcination thereof at a temperature exceeding 450 °C.

**3.** Process according to claim 2, characterized in that the titanozeosilite used is obtained by a process having the following stages:
(i) preparation of a reaction mixture in an aqueous medium containing at least one silicon dioxide source, a titanium dioxide source, fluoride ions and a structuring agent, with molar Ti/Si ratios between 1.5 and 0.002, F/Si molar ratios between 10 and 0.04, $H_2O$/Si molar ratios between 400 and 4 and structuring agent/Si molar ratios between 2 and 0.02 and a reaction mixture pH between approximately 1.5 and approximately 10.5,
(ii) crystallization of this reaction mixture and the recovery of the crystalline precipitate and
(iii) calcination of the latter at a temperature exceeding 450 °C.

**4.** Process according to claim 3, characterized in that during the preparation of the titanozeosilite the Ti/Si molar ratio in the reaction mixture is between 1 and 0.01.

**5.** Process according to one of the claims 3 or 4, characterized in that during the preparation of the titanozeosilite the F/Si molar ratio in the reaction mixture is between 6 and 0.06.

**6.** Process according to one of the claims 3 to 5, characterized in that during the preparation of the titanozeosilite the $H_2O$/Si molar ratio in the reaction mixture is between 100 and 6.

**7.** Process according to one of the claims 3 to 6, characterized in that during the preparation of the titanozeosilite the molar ratio between the structuring agent and the Si is between 1 and 0.04.

**8.** Process according to one of the claims 1 to 7, characterized in that the phenol/hydrogen peroxide molar ratio is 25/1 to 3/1 and preferably 20/1 to 4/1.

**9.** Process according to one of the claims 1 to 8 performed discontinuously, characterized in that the catalyst represents by weight based on the weight of the compound of formula (I) used 0.1 to 20% and preferably 0.5 to 10%.

**10.** Process according to one of the claims 1 to 9, characterized in that the process is performed continuously on a fixed catalyst bed.

**11.** Process according to one of the claims 1 to 10, characterized in that the hydrogen peroxide is used in the form of an aqueous solution.

**12.** Process according to one of the claims 1 to 11, characterized in that the hydrogen peroxide is used in the form of an organic solution.

**13.** Process according to one of the claims 1 to 12, characterized in that the hydroxylation reaction is performed in a solvent of the compound of formula (I), which is preferably miscible or partly miscible with water, such as water, an alcohol, a ketone, a nitrile, a carboxylic acid, an ester, an ether or a polar aprotic solvent.

**14.** Process according to one of the claims 1 to 13, characterized in that the hydroxylation reaction is performed at a temperature between 45 and 160 °C.

**15.** Process according to one of the claims 1 to 14, characterized in that it is applied to phenols and phenol ethers of formula (I) in which $R_5$ represents a hydrogen atom, a methyl group or an ethyl group and $R_6$ represents a hydrogen atom, a methyl, ethyl or tert. butyl group, or a methoxy or ethoxy group.

**16.** Process according to one of the claims 1 to 15, characterized in that it is applied to phenols and phenol ethers chosen from among phenol, anisole, orthocresol, metacresol, paracresol, 4 tert. butyl phenol, 2-methoxy phenol and 4-methoxy phenol.

**Patentansprüche**

**1.** Verfahren zur Hydroxylierung von Phenolen oder Phenolethern der allgemeinen Formel (I)

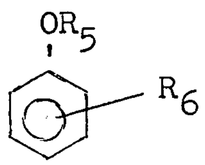

(I)

worin

$R_5$ ein Wasserstoffatom, eine Methylgruppe, eine Ethylgruppe oder eine Phenylgruppe bedeutet,

$R_6$ ein Wasserstoffatom, einen Alkylrest mit 1 bis 4 Kohlenstoffatomen, einen Alkoxyrest mit 1 bis 4 Kohlenstoffatomen, einen Phenyl- oder Cyclohexylrest wiedergibt,

durch Umsetzung mit Wasserstoffperoxid, dadurch gekennzeichnet, daß die Reaktion in Anwesenheit einer wirksamen Menge eines Zeolithen mit MFI-Struktur auf Basis von Siliciumoxid und Titanoxid durchgeführt wird:

- der nach der Calcinierung die folgende Formel (II)

  $Si_{(96-x)}, Ti_x O_{192}$ (II)

  besitzt, worin x zwischen etwa 0,1 und 6 beträgt,
- der zwischen 0,01 und 0,8% Fluor, auf das Gewicht bezogen, nach der Calcinierung enthält,
- und der ein monoklines, kristallines System aufweist.

**2.** Verfahren zur Hydroxylierung von Phenolen oder Phenolethern der allgemeinen Formel (I)

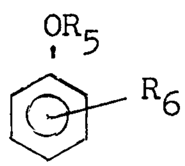

(I)

worin

$R_5$ ein Wasserstoffatom, eine Methylgruppe, eine Ethylgruppe oder eine Phenylgruppe bedeutet,

$R_6$ für ein Wasserstoffatom, einen Alkylrest mit 1 bis 4 Kohlenstoffatomen, einen Alkoxyrest mit 1 bis 4 Kohlenstoffatomen, einen Phenyl- oder Cyclohexylrest steht,

durch Umsetzung mit Wasserstoffperoxid, dadurch gekennzeichnet, daß die Reaktion in Anwesenheit einer wirksamen Menge eines Zeolithen mit MFI-Struktur auf Basis von Siliciumoxid und Titanoid durchgeführt wird, der nach der Calcinierung die folgende Formel (II)

$Si_{(96-x)}, Ti_x O_{192}$ (II)

aufweist, worin x zwischen etwa O,1 und 6 beträgt, erhalten durch ein Verfahren, das die folgenden Stufen aufweist:

(i) die Herstellung eines Reaktionsgemisches in wäßrigem Milieu, das zumindest eine Quelle für das Siliciumoxid, eine Quelle für das Titanoxid, Fluoridionen und ein Struktur verleihendes Mittel enthält, wobei der pH des Reaktionsgemisches zwischen etwa 1,5 und etwa 10,5 beträgt,

(ii) die Kristallisation dieses Reaktionsgemisches und die Gewinnung des kristallinen Niederschlags und

(iii) die Calcinierung desselben bei einer Temperatur von höher als 450 °C.

**3.** Verfahren gemäß Anspruch 2, dadurch gekennzeichnet, daß der verwendete Titanozeosilit durch ein Verfahren erhalten wird, das die folgenden Stufen aufweist:

(i) die Herstellung eines Reaktionsgemisches in wäßrigem Milieu, das zumindest eine Quelle für das Siliciumoxid, eine Quelle für das Titanoxid, Fluoridionen und ein Struktur verleihendes Mittel enthält, wobei die Molverhältnisse Ti/Si zwischen 1,5 und 0,002, F/Si zwischen 10 und 0,04, $H_2O$/Si zwischen 400 und 4, Struktur verleihendes Mittel/Si zwischen 2 und 0,02 liegen und der pH des Reaktionsgemisches zwischen etwa 1,5 und etwa 10,5 beträgt,

(ii) die Kristallisation dieses Reaktionsgemisches und die Gewinnung des kristallinen Niederschlags und

(iii) die Calcinierung desselben bei einer Temperatur von höher als 450 °C.

**4.** Verfahren gemäß Anspruch 5, dadurch gekennzeichnet, daß bei der Herstellung des Titanozeosiliten das Molverhältnis Ti/Si in dem Reaktionsgemisch zwischen 1 und 0,01 liegt.

**5.** Verfahren gemäß einem der Ansprüche 3 oder 4, dadurch gekennzeichnet, daß bei der Herstellung des Titanozeosiliten das Molverhältnis F/Si in dem Reaktionsgemisch zwischen 6 und 0,06 beträgt.

**6.** Verfahren gemäß einem der Ansprüche 3 bis 5, dadurch gekennzeichnet, daß bei der Herstellung des Titanozeosiliten das Molverhältnis $H_2O$/Si in dem Reaktionsgemisch zwischen 100 und 6 beträgt.

**7.** Verfahren gemäß einem der Ansprüche 3 bis 6, dadurch gekennzeichnet, daß bei der Herstellung des Titanozeosiliten das Molverhältnis Struktur verleihendes Mittel/Si zwischen 1 und 0,04 beträgt.

**8.** Verfahren gemäß einem der Ansprüche 1 bis 7, dadurch gekennzeichnet, daß das Molverhältnis Phenol/Wasserstoffperoxid 25/1 bis 3/1 und vorzugsweise 20/1 bis 4/1 beträgt.

**9.** Verfahren gemäß einem der Ansprüche 1 bis 8, diskontinuierlich durchgeführt, dadurch gekennzeichnet, daß der Katalysator, ausgedrückt als Gewicht, bezogen auf das Gewicht der eingesetzten Verbindung der Formel (I), 0,1 bis 20% und vorzugsweise 0,5 bis 10% beträgt.

**10.** Verfahren gemäß einem der Ansprüche 1 bis 9, dadurch gekennzeichnet, daß das Verfahren kontinuierlich über einem Katalysatorfestbett durchgeführt wird.

**11.** Verfahren gemäß einem der Ansprüche 1 bis 10, dadurch gekennzeichnet, daß das Wasserstoffperoxid in Form einer wäßrigen Lösung eingesetzt wird.

**12.** Verfahren gemäß einem der Ansprüche 1 bis 11, dadurch gekennzeichnet, daß das Wasserstoffperoxid in Form einer organischen Lösung eingesetzt wird.

**13.** Verfahren gemäß einem der Ansprüche 1 bis 12, dadurch gekennzeichnet, daß die Hydroxylierungsreaktion in einem Lösungsmittel der Verbindung der Formel (I), das vorzugsweise mit Wasser mischbar oder teilweise mischbar ist, wie Wasser, ein Alkohol, ein Keton, ein Nitril, eine Carbonsäure, ein Ester, ein Ether oder ein polares aprotisches Lösungsmittel, durchgeführt wird.

**14.** Verfahren gemäß einem der Ansprüche 1 bis 13, dadurch gekennzeichnet, daß die Hydroxylierungsreaktion bei einer Temperatur zwischen 45 und 160 °C durchgeführt wird.

**15.** Verfahren gemäß einem der Ansprüche 1 bis 14, dadurch gekennzeichnet, daß es auf Phenole oder Phenolether der Formel (I) angewandt wird, worin $R_5$ ein Wasserstoffatom, eine Methylgruppe oder eine Ethylgruppe bedeutet und $R_6$ ein Wasserstoffatom, eine Methyl-, Ethyl- oder tert.-Butylgruppe, eine Methoxy- oder Ethoxygruppe wiedergibt.

**16.** Verfahren gemäß einem der Ansprüche 1 bis 15, dadurch gekennzeichnet, daß es auf Phenole und Phenolether angewandt wird, ausgewählt unter Phenol, Anisol, Orthocresol, Metacresol, Paracresol, 4-tert.-Butylphenol, 2-Methoxyphenol, 4-Methoxyphenol.